# EUROPÄISCHE PATENTANMELDUNG

(11) **EP 2 366 680 A2**
(43) Veröffentlichungstag der Anmeldung: **21.09.2011**
(21) Anmeldenummer: 11158849.7
(22) Anmeldetag: 18.03.2011
(51) Int. Cl.: C07B 61/00, C07C 67/08, C07C 67/343, C07C 69/80, C07C 69/76

(54) **Verfahren zur Herstellung von 4'-Halogenalkyl-biphenyl-2-carbonsäuren**

(30) Priorität: 20.03.2010 DE 102010012133
(71) Anmelder: Saltigo GmbH, 40764 Langenfeld (DE)
(72) Erfinder: Kreis, Michael, 51373 Leverkusen (DE); Cotté, Alain, 51377 Leverkusen (DE); Steinhaus, Antje, 50733 Köln (DE); Gooßen, Lukas, 67663 Kaiserslautern (DE); Knauber, Thomas, 67663 Kaiserslautern (DE)
(74) Vertreter: Pettrich, Klaus-Günter

(57) **Zusammenfassung**

Die Erfindung betrifft ein Verfahren zur Herstellung gegebenenfalls substituierter 4'-Halogenalkyl-biphenyl-2-carbonsäureester und ein Verfahren zur Herstellung korrespondierender gegebenenfalls substituierter 4'-Halogenalkyl-biphenyl-2-carbonsäuren durch metalkatalysierte decarboxylierende Kreuzkupplung in Gegenwart mindestens einer Kupferquelle, mindestens einem zyklischen, chelatisierenden Amin, mindestens einem Phosphinliganden und mindestens einer Palladiumquelle.

## Beschreibung

Die Erfindung betrifft ein Verfahren zur Herstellung gegebenenfalls substituierter 4'-Halogenalkyl-biphenyl-2-carbonsäureester und ein Verfahren zur Herstellung korrespondierender gegebenenfalls substituierter 4'-Halogenalkyl-biphenyl-2-carbonsäuren.

4'-Halogenalkyl-biphenyl-2-carbonsäuren stellen wichtige Zwischenprodukte für die Herstellung von Arzneimitteln dar. Insbesondere ist 4'-Trifluormethyl-biphenyl-2-carbonsäure selbst als pharmazeutischer Wirkstoff unter dem Namen Xenalipin bekannt. Xenalipin senkt den Cholesteringehalt und den Anteil an Trigylceride im Blutplasma (Atherosclerosis, 1987, 64, 27-35). Daher stellt 4'-Trifluormethyl-biphenyl-2-carbonsäure ein Arzneimittel zur Prävention von Herz-und Kreislauferkrankungen dar. Die 4'-Halogenalkyl-biphenyl-2-carbonsäuren lassen sich in an sich bekannter Weise durch Verseifung von 4'-Halogenalkyl-biphenyl-2-carbonsäureestern herstellen.

Aus JP 2004-067595 A ist ein Verfahren zur Herstellung eines 4'-Halogenalkyl-biphenyl-2-carbonsäureesters, insbesondere des 4'-Trifluormethyl-biphenyl-2-carbonsäureisopropylesters, durch Nickel katalysierter Kopplung von 2-Chlorbenzoesäure-isopropylester und 4-Chlorbenzotrifluorid, bekannt. Dieses Verfahren hat den Nachteil, dass stöchiometrische Mengen an Nickel eingesetzt werden müssen und lediglich geringe Ausbeuten erreicht werden.

Verfahren zur Herstellung von 4'-Halogenalkyl-biphenyl-2-carbonsäuren, insbesondere der 4'-Trifluormethyl-biphenyl-2-carbonsäure, mittels Suzuki-Miyaura-Kupplung von Arylboronsäure mit den korrespondierenden Aryliodiden, Arylbromiden oder Arylchloriden sind aus DE 19963563 A, Tetrahedron Letters, 47, 2006, 4225-4229 und Synthesis, 8, 2002, 1043-1046 bekannt. Besonders für industrielle Anwendungen hat diese Herangehensweise jedoch gravierende Nachteile. Die Boronsäuren werden in aufwändigen Synthesen aus hochreaktiven, luft- und feuchtigkeitslabilen Organometallverbindungen (z.B. Grignardreagenzien oder Lithiumorganyle) und Trialkylboraten hergestellt wobei viele funktionelle Gruppen nicht toleriert werden. Darunter befinden sich auch Carbonsäuren und deren Derivate. Das Verfahren ist daher nicht ökonomisch in technischen Prozessen einsetzbar und ist zudem sicherheitstechnisch bedenklich.

Weitere Verfahren zur Herstellung von 4'-Halogenalkyl-biphenyl-2-carbonsäuren durch katalytische Kreuzkupplungen von Arylhalogeniden mit Diborverbindungen sind aus CN 1944386 und EP 1122234 A bekannt. Nachteilig bei diesen Verfahren ist, dass sicherheitstechnisch bedenkliche Edukte eingesetzt werden, die Verfahren mindestens vier Verfahrensschritte umfassen und lediglich geringe Ausbeuten erzielt werden.

EP 1223158 beschreibt ein Verfahren zur Herstellung von 4'-Halogenalkyl-biphenyl-2-carbonsäuren, insbesondere der 4'-Trifluormethyl-biphenyl-2-carbonsäure, ausgehend von 2-Cyano-4-methylbiphenyl über Chlorierung, Fluorierung und anschließender Verseifung. Nachteil dieses Verfahrens ist, dass die Edukte aufwändig hergestellt werden müssen und dass das Verfahren ineffizient in technischen Prozessen verläuft.

Ein vierstufiges Verfahren zur Herstellung von 4'-Trifluormethyl-biphenyl-2-carbonsäure über eine Negishi-Kupplung ausgehend von Benzoesäure ist aus Journal of Labelled Compounds and Radiopharmaceuticals 31, 1992, 1011-1017 und aus Journal Organic Preparations and Procedures International 27, 1995, 367-372 bekannt. Das Verfahren ist aufgrund der eingesetzten hochreaktiven Organolithium-Verbindung sicherheitstechnisch bedenklich und macht den Einsatz von 2-Phenyl-4,4-dimethyl-2-oxazolin als geschütztes und teures Benzoesäureäquivalent nötig, so dass das Verfahren zudem nicht ökonomisch in technischen Prozessen eingesetzt werden kann.

Aus US 4,578,522 B ist bekannt, dass 4'-Trifluormethyl-biphenyl-2-carbonsäure ausgehend von 2-(2-Methoxyphenyl)-4,4-dimethyl-2-oxazolin und 4-Brombenzotrifluorid in einer Wurtz-Grignard-Reaktion mit anschließender Verseifung hergestellt werden kann. Nachteilig bei diesem Verfahren ist, dass lediglich geringe Ausbeuten erzielt werden.

Sämtlichen Verfahren ist gemeinsam, dass sie entweder aus sicherheitstechnischen und ökonomischen Gründen ungeeignet sind oder das Zielmolekül nur über vielstufige Synthesen darzustellen ist und lediglich geringe Ausbeuten erzielt werden.

Es bestand daher weiterhin ein Bedürfnis nach einem Verfahren zur Herstellung von gegebenenfalls substituierten 4'-Halogenalkyl-biphenyl-2-carbonsäuren mit dem die Nachteile des Standes der Technik überwunden werden können und mit dem gegebenenfalls substituierte 4-Halogenalkyl-2'-biphenylcarbonsäuren in guten Ausbeuten und effizient in technischen Prozessen hergestellt werden können.

Überraschend wurde ein Verfahren gefunden indem zunächst die 4'-Halogenalkyl-biphenyl-2-carbonsäureester durch metalkatalysierte decarboxylierende Kreuzkupplung ausgehend von gegebenenfalls substituiertem Phtalsäureanhydrid in guten Ausbeuten und hohen Reinheiten hergestellt werden können. Die 4'-Halogenalkyl-biphenyl-2-carbonsäureester können dann durch Verseifung in die 4'-Halogenalkyl-biphenyl-2-carbonsäuren überführt werden.

Die Erfindung betrifft daher ein Verfahren zur Herstellung der Verbindungen der Formel (I) 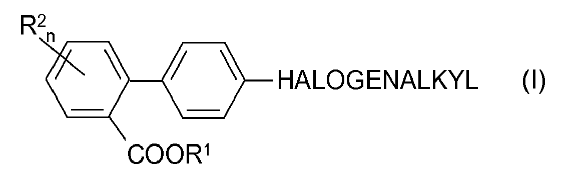 wobei R¹ für C₁-C₆-Alkyl, C₆-C₂₄-Aryl oder C₇-C₁₅-Arylalkyl steht und R² unabhängig voneinander oder gleich sein kann und für C₁-C₆-Alkyl, C₂-C₆-Alkenyl, C₃-C₁₆-Heteroaryl, C₁-C₆-Alkoxy, C₁-C₆-Aryloxy, C₁-C₆-Acyl, C₇-C₁₅-Arylalkyl, C₁-C₈-Mono- und Dialkylamino, Nitro, Cyano, C₁-C₆-Alkylthio, C₆-C₂₄-Aryl, einen 3- bis 7-gliedrigen gesättigten oder teilweise ungesättigten Heterozyklus oder Wasserstoff steht und im Falle, dass R² ein C₃-Heteroaryl darstellt, das C₃-Heteroaryl drei Kohlenstoffatome und mindestens zwei Stickstoffatome oder ein Stickstoffatom und ein Sauerstoffatom oder ein Stickstoffatom und ein Schwefelatom enthält und n = 1, 2, 3 oder 4 ist und HALOGENALKYL für einen einfach, mehrfach oder vollständig halogenierten, also durch F, Cl, Br, oder/und I substituierten, geradkettigen, zyklischen oder verzweigten C₁-C₆-Alkylrest steht, bei dem Verbindungen der Formel (II) 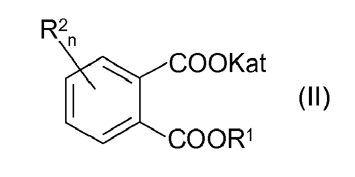 worin R¹, R² und n die vorgenannte Bedeutung besitzen und Kat ein anorganisches oder organisches einfach geladenes Kation darstellt,
in Gegenwart mindestens einer Kupferquelle,
in Gegenwart von mindestens einem zyklischen, chelatisierenden Amin
in Gegenwart von mindestens einem Phosphinliganden und
in Gegenwart mindestens einer Palladiumquelle
mit Verbindungen der Formel (III) 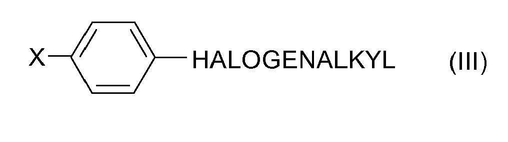 in denen HALOGENALKYL die vorgenannte Bedeutung besitzt und X für Halogen = Cl, Br, oder I, Pseudohalogen, Arylsulfonat oder Alkylsulfonat steht, zu Verbindungen der Formel (I) umgesetzt werden.

Bevorzugt steht R¹ für Methyl, Ethyl, s-Propyl, n-Propyl, n-, s-, i-, tert-Butyl, neo-Pentyl, cyclo-Hexyl, Benzyl oder Phenyl. Ganz besonders bevorzugt steht R¹ für iso-Propyl bzw. s-Propyl.

Bevorzugt steht R² für C₁-C₆-Alkyl, C₂-C₆-Alkenyl, C₃-C₁₆-Heteroaryl, C₁-C₆-Acyl, C₆-C₂₄-Aryl oder/und Wasserstoff. Besonders bevorzugt steht R² für Methyl, Ethyl, s-, n-Propyl, n-, i-, s-, tert.-Butyl oder/und Wasserstoff. Ganz besonders bevorzugt steht R² für Wasserstoff.
n ist bevorzugt 1 oder 2, ganz besonders bevorzugt ist n = 1. Falls n = 1 ist, steht R² bevorzugt für C₁-C₆-Alkyl, C₂-C₆-Alkenyl, C₃-C₁₆-Heteroaryl, C₆-C₂₄-Aryl , C₁-C₆-Acyl, oder/und Wasserstoff. Ganz besonders bevorzugt stellt R² dann einen C₁-C₆-Alkyl, C₂-C₆-Alkenyl oder/und Wasserstoff dar.

Falls n = 2, 3 oder 4 ist, kann R² gleich oder verschieden sein und steht dann bevorzugt für C₁-C₆-Alkyl, C₂-C₆-Alkenyl, C₃-C₁₆-Heteroaryl, C₆-C₂₄-Aryl , C₁-C₆-Acyl, oder/und Wasserstoff. Ganz besonders bevorzugt stellt R² dann einen C₁-C₆-Alkyl, C₂-C₆-Alkenyl oder/und Wasserstoff dar.

HALOGENALKYL steht für einen C₁-C₆-Halogenalkyl. HALOGENALKYL steht vorzugsweise für Dichlormethyl, Difluormethyl, Chlordifluormethyl, Fluormethyl, 2,2,2-Trifluorethyl, Difluorethyl, Fluorethyl, Fluorpropyl, Tetrafluorethyl, Heptafluorpropyl oder für einen C₁-C₆-Perhalogenalkyl, der einen vollständig durch Halogenatome substituierten C₁-C₆-Alkylrest darstellt. Beispielhaft und vorzugsweise steht C₁-C₆-Perhalogenalkyl für Trifluormethyl, Trichlormethyl, Tribrommethyl, Pentafluorethyl, Heptafluorpropyl, cylco-Nonafluorpentyl, cyclo-Nonachlorcylcopentyl, Heptafluorisopropyl und Nonafluorbutyl. Bevorzugt steht HALOGENALKYL für ein C₁-C₆-Perfluoralkyl. Bevorzugt steht C₁-C₆-Perfluoralkyl für Difluormethyl, Trifluormethyl, Pentafluorehtyl, Heptafluorisopropyl und Nonafluorbutyl. Besonders bevorzugt steht HALOGENALKYL oder/und C₁-C₆- Perhalogenalkyl für Trifluormethyl, Pentafluorethyl, Trichlormethyl, Tribrommethyl oder Pentachlorethyl. Ganz besonders bevorzugt steht HALOGENALKYL für Trifluormethyl, Pentafluorethyl oder Heptafluorisopropyl.

Kat stellt bevorzugt ein Alkalimetalkation oder ein organisches Kation aus der Reihe Ammonium, Pyridinium oder Phosphonium dar. Besonders bevorzugt steht Kat für ein Kaliumkation.

Als Kupferquellen werden anorganische oder organische Kupfer(I) oder Kupfer(II)-Verbindungen oder elementares Kupfer eingesetzt. Als Kupfer(I)verbindungen oder Kupfer(II)verbindungen können Kupfer(I)halogenide, wie z.B. Kupfer(I)bromid oder Kupfer(I)chlorid oder Kupfer(II)halogenide, wie z.B. Kupfer(II)bromid oder Kupfer(II)chlorid oder Kupfer(I)pseudohalogenide, wie z.B. Kupfer(I)thiocyanat, Kupfer(I)isothiocyanat, Kupfer(I)isocyanat oder Kupfer(I)cyanat oder Kupfer(II)pseudohalogenide, wie z.B. Kupfer(II)thiocyanat, Kupfer(II)isothiocyanat oder Kupfer(II)cyanat oder Kupfer(I)cyanid oder Kupfer(II)cyanid oder organische Kupfer(I)komplexe oder organische Kupfer(II)komplexe, wie z.B. Dichlor(1,10-phenanthroline)kupfer(II) Dibrom(1,10-phenanthroline)kupfer(II) oder Kupfer(II)diaminkomplexe, wie z.B. Di-µ-hydroxy-bis(N,N,N',N'-tetramethylethylendiamin)kupfer(II)chlorid oder Kupfer(I)oxid oder Kupfer(II)oxid oder Gemische dieser Verbindungen eingesetzt. Bevorzugt werden als Kupferquellen Kupfer(I)halogenide, Kupfer(II)halogenide oder Kupfer(II)oxid eingesetzt. Ganz besonders bevorzugt wird als Kupferquelle Kupfer(I)chlorid oder Kupfer(II)oxid eingesetzt.

Als Paladiumquellen werden anorganische oder organische Pd(I)- oder Pd(II)-Verbindungen oder elementares Pd eingesetzt. Besonders bevorzugt werden Palladium(II)verbindungen und noch weiter bevorzugt, Palladium(II)sulfat, Palladium(II)halogenide, wie Palladium(II)bromid oder Palladium(II)chlorid, Palladium(II)pseudoha**logenide**, **wie** Palladium(II)thiocyanat, Palladium(II)isothiocyanat Palladium(II)cyanat oder Palladium(II)isocyanat, oder Palladium(II)cyanid, oder organische Palladium(II)verbindungen, wie Palladium(II)carboxylat, wie Palladium(II)acetat, Palladium(II)formiat, Palladium(II)propylat oder Palladium(II)butylat oder Palladium(II)acetylacetonat, oder Palladium(II)-komplexe wie Dichlor(N,N,N;N'-tetramethylethylenediamin)palladium(II), Dichlor(1, 10-phenanthroline)palladium(II), Bis(tri-phenylphosphine)palladium(II), Bis(benzonitril)palladium(11), 1,4-Bis(diphenylphosphino)butan-palladium(II)chloride, (2,2'-Bipyridin)dichlorpalladium(II) , (1 , 3-Bis(diphenylphosphino)-propan)palladium(II)chloride, oder Gemische dieser Verbindungen eingesetzt. Ganz besonders bevorzugt wird als Palladiumquelle Palladium(11)acetylacetonat eingesetzt.

Als chelatisierende, zyklische Amine können beispielsweise und vorzugsweise substituierte, zyklische aromatische Diamine, wie z.B. Phenantrolin, Bipyridin Terpyridin und substiuierte Derivate dieser Verbindungen eingesetzt werden. Besonders bevorzugt werden als zyklische, chelatisierende Amine Phenantrolin, Bipyridin, Terpyridin und substituierte Derivate dieser Verbindungen eingesetzt. Ganz besonders bevorzugt wird 1, 10-Phenanthrolin als zyklisches, chelatisierendes Amin eingesetzt.

Als Phosphinliganden werden Verbindungen der Formel (IV) eingesetzt, 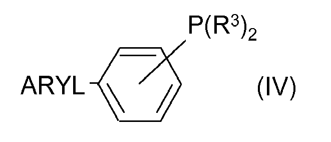 wobei ARYL einen einfach oder mehrfach unabhängig voneinander durch C₁-C₆-Alkyl, C₁-C₆-Alkoxy, Sulfonaten oder C₁-C₈-Mono- bzw. Dialkylamino substituierten oder unsubstituierten C₆-C₂₄-Aryl darstellt und R³ einen C₁-C₆-Alkylrest oder einen einfach oder mehrfach substiuierten oder unsubstituierten C₆-C₂₄-Aryl darstellt.

Bevorzugt steht ARYL für einen Phenylrest der gegebenenfalls, unabhängig voneinander mehrfach substituiert sein kann durch C₁-C₆-Alkyl, C₁-C₆-Alkoxy, Sulfonaten oder C₁-C₈-Mono- bzw. Dialkylamino. Ganz besonders bevorzugt steht ARYL für einen Phenylrest der einfach oder mehrfach durch Methoxy, tert.-Butyl, Sulfonaten oder Dimethylamino substituiert sein kann.

R³ steht bevorzugt für einen Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, Benzyl, o-,p-,m-Tolyl, o-,p-,m-Hydroxytolyl oder o-,p,-m-Methoxybenzyl. Besonders bevorzugt steht R³ für tert.-Butyl oder Cyclohexyl.

Ganz besonders bevorzugt stehen die Phosphinliganden für 2-(Di-*tert*-butyl-phosphino)-biphenyl, Dicyclohexylphosphin-2-biphenyl, 2-Dicydohexylphosphino-2'-(N,N-dimethylamino)biphenyl 2-Dicyclohexylphosphino-2',6'-dimethoxybiphenyl, 2-Dicyclohexylphosphino-2',4',6'-triisopropyl-biphenyl, Dicyclohexylphosphino-2'-methylbiphenyl 2-Di-*tert*-butylphosphino-2',4',6'-triisopropyl-biphenyl, 2-Di-*tert*-butylphosphino-3,4,5,6-tetramethyl-2',4',6'-triisopropyl-1,1'-biphenyl, 2-Di-*tert-*butylphosphino-2'-methylbiphenyl, 2-Di-*tert*-butylphosphino-2'-(*N,N*-dimethylamino)biphenyl, 2-Dicyclohexylphosphino-2'-(*N,N*-dimethylamino)biphenyl, 2-(Dicyclohexylphosphino)biphenyl, 2-(Di-tert-butylphosphino)biphenyl, Natrium-2-dicyclohexylphosphino-2',6'-dimethoxybiphenyl-3'-sulfonat, 2-Diphenylphosphino-2'-(*N,N*-dimethylamino)biphenyl oder 2-Dicyclohexylphosphino-2',6'-diisopropoxybiphenyl.

X steht bevorzugt für Cl, Br, I, F, N₂⁺, Triflat, Tosylat, Nonaflat oder Mesylat. Besonders bevorzugt steht X für Cl, Triflat, Tosylat, Nonaflat oder Mesylat. Ganz besonders bevorzugt steht X für Cl.

Als besonders bevorzugt hat sich der Einsatz eines Kupfer(II)oxid mit einem 1,10-Phenanthrolin in Kombination mit einem Palladium(II)acetylacetonat und einem Phosphinliganden herausgestellt. Insbesondere ist weiter bevorzugt, dass als Phosphinligand ein sterisch, anspruchsvoller, elektronenreicher Phosphinligand, wie z.B. 2-Dicyclohexylphosphinobiphenyl, eingesetzt wird.

Der Rahmen der Erfindung erfasst alle oben stehenden und im Folgenden aufgeführten allgemeinen oder in Vorzugsbereichen genannten Restedefinitionen, Parameter und Erläuterungen untereinander, also auch zwischen den jeweiligen Bereichen und Vorzugsbereichen in beliebiger Kombination. Alkyl beziehungsweise Alkenyl beziehungsweise Alkoxy steht jeweils unabhängig für einen geradkettigen, zyclischen oder verzweigten Alkyl- beziehungsweise Akenyl- beziehungsweise AlkoxyRest. Gleiches gilt für den nichtaromatischen Teil eines Arylalkyl-Restes.

C₁-C₆-Alkyl steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und 1-Ethyl-2-methylpropyl. Bevorzugt steht C₁-C₆-Alkyl für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl und n-Hexyl.

Beispielhaft und vorzugsweise steht C₂-C₆-Alkenyl für Vinyl, Allyl, Isopropenyl und n-But-2-en-1-yl.

C₁-C₆-Alkoxy steht beispielsweise für Methoxy, Ethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy und n-Hexoxy.

C₁-C₆-Alkylthio steht für einen geradkettigen, zyklischen oder verzweigten Alkylthiorest mit 1 bis 6 Kohlenstoffatomen. Bevorzugt ist ein geradkettiger oder verzweigter Alkylthiorest mit 1 bis 4, besonders bevorzugt mit 1 bis 3 Kohlenstoffatomen. Beispielsweise und vorzugsweise seien genannt: Methylthio, Ethylthio, n-Propylthio, Isopropylthio, tert.-Butylthio, n-Pentylthio und n-Hexylthio.

C₆-C₂₄-Aryl steht im Rahmen der Erfindung für einen mono-, bi- oder trizyklischen carbozyklischen aromatischen Rest mit vorzugsweise 6 bis 24 aromatischen Kohlenstoffatomen. Weiterhin können die carbozyklischen aromatischen Reste mit bis zu fünf gleichen oder verschiedenen Substituenten pro Zyklus substituiert sein, ausgewählt aus der Gruppe C₁-C₆-Alkyl, C-₇-C₁₅-Arylalkyl, C₆-C₂₄-Aryl, C₁-C₈-Mono- und Dialkylamino, Nitro, Cyano oder 3- bis 7-gliedriger gesättigter oder teilweise ungesättigter Heterozyklus. Beispielhaft und bevorzugt steht Aryl für Biphenyl, Phenyl, o-, m-, p-Tolyl, Naphthyl, Phenanthrenyl, Anthracenyl, Acetnaphtylen und Fluorenyl.

C₇-C₁₅-Arylalkyl bedeutet jeweils unabhängig voneinander einen geradkettigen, zyklischen, oder verzweigten Alkyl-Rest nach vorstehender Definition, der einfach, mehrfach oder vollständig durch Aryl-Reste gemäß vorstehender Definition substituiert sein kann. Beispielsweise und vorzugsweise steht C₇-C₁₅-Arylalkyl für Benzyl.

Arylsulfonate stehen im Rahmen der Erfindung für einen C₆-C₂₄-Arylrest oder für einen C₇-C₁₅-Arylalkylrest nach vorgenannter Definition der durch eine oder mehrer Sulfonsäuregruppe substituiert ist und über ein Sauerstoffatom der Sulfonsäuregruppe verknüpft ist. Beispielsweise und vorzugsweise stehen Arylsulfonate für Tosylat oder Benzylat.

Alkylsulfonate stellen im Rahmen der Erfindung für einen C₁-C₆-Alkylrest nach vorgenannter Definition der durch eine oder mehrer Sulfonsäuregruppen substituiert ist und über ein Sauerstoffatom der Sulfonsäuregruppe verknüpft ist. Beispielsweise und vorzugsweise stehen Alkylsulfonate für Mesylat, Triflat oder Nonaflat eingesetzt.

3- bis 7-gliedriger gesättigter oder teilweise ungesättigter Heterozyklus steht im Rahmen der Erfindung für einen Heterozyklus, mit bis zu 3 gleichen oder verschiedenen Heteroatomen aus der Reihe S, N und/oder O, der über ein Ringkohlenstoffatom oder ein Ringstickstoffatom verknüpft ist und der eine oder zwei Doppelbindungen enthalten kann. Bevorzugt ist ein 5- bis 7-gliedriger gesättigter Heterozyklus mit bis zu 2 gleichen oder verschiedenen Heteroatomen aus der Reihe S, N und/oder O. Beispielhaft seien genannt: Tetrahydrofur-2-yl, Tetrahydrofur-3-yl, Pyrrolidin-1-yl, Pyrrolidin-2-yl, Pyrrolidin-3-yl, Pyrrolin-1-yl, Piperidin-1-yl, Piperidin-4-yl, 1,2-Dihydropyridin-1-yl, 1,4-Dihydropyridin-1-yl, Piperazin-1-yl, Morpholin-4-yl, Thiomorpholin-4-yl, Azepin-1-yl, 1,4-Diazepin-1-yl. Bevorzugt sind Piperidinyl, Piperazinyl, Morpholinyl und Pyrrolidinyl.

C₃-C₁₆-Heteroaryl steht im Rahmen der Erfindung für einen aromatischen Heterozyklus, mit bis zu 3 gleichen oder verschiedenen Heteroatomen aus der Reihe S, N und/oder O, der über ein Ringkohlenstoffatom des Heteroaromaten, gegebenenfalls auch über ein Ringstickstoffatom des Heteroaromaten verknüpft ist und der zwischen 3 und 16 Kohlenstoffatome (C₃-C₁₆-Heteroaryl), vorzugsweise 3 bis 7 (C₃-C₇) Kohlenstoffatome und besonders bevorzugt 4 bis 5 (C₄-C₅) Kohlenstoffatome (C₄-C₅-Heteroaryl) aufweist. C₃-C₁₆-Heteroaryl, C₃-C₇-Heteroaryl, und C₃-C₅-Heteroaryl weisen immer mindestens so viele Heteroatome auf, dass der Heteroaromat aromatisch ist. Ein C₃-N-Heteroaryl weist also drei Kohlenstoffatome und mindestens zwei Stickstoffatome auf. C₃-C₁₆-Heteroaryl kann weiter substituiert sein durch Reste ausgewählt aus der Gruppe C₁-C₆-Alkyl, C₁-C₆-Alkoxy, C₇-C₁₅-Arylakyl, C₁-C₈-Mono- und Dialkylamino, Nitro, Cyano, C₁-C₆-Alkylthio, C₆-C₂₄-Aryl oder 3- bis 7-gliedriger gesättigter oder teilweise ungesättigter Heterozyklus. Beispielhaft und vorzugsweise seien als C₃-C₁₆₋Heteroaryl genannt: Pyridyl, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl, Benzofuranyl oder Dibenzofuranyl.

C₁-C₈-Mono- bzw. Dialkylamino bzw. steht im Rahmen der Erfindung für eine Amino-Gruppe die mit einem oder zwei gleichen oder verschiedenen, zyklischen, geradkettigen oder verzweigten Alkylsubstituenten, die vorzugsweise jeweils 1 bis 8 Kohlenstoffatomen aufweisen.

Beispielhaft und vorzugsweise steht C₁-C₈-Monoalkylamino für Methylamino, Ethylamino, n-Propylamino, Isopropylamino, t-Butylamino, n-Pentylamino und n-Hexylamino.

Beispielhaft und vorzugsweise steht C₁-C₈-Dialkylamino für *N,N*-Dimethylamino, *N,N*-Diethylamino, *N-*Ethyl-*N*-methylamino, *N*-Methyl-*N*-n-propylamino, *N*-Isopropyl-*N-*n-propylamino, *N-*t*-*Butyl-N methylamino, *N*-Ethyl-*N-*n-pentylamino und *N*-n-Hexyl-*N*-methylamino.

Pseudohalogen bezeichnet im Rahmen der Erfindung Substituenten, die in ihren chemischen Eigenschaften eine große Ähnlichkeit zu den Halogenen aufweisen. Dies sind z.B. Sulfonate und Halogensulfonate, wie z.B. Tosylat, Triflat und Nonafluorbutylsulfonat, aber auch Thiocyanat, Cyanat, Isothiocyanat, Isocyanat und Azid. Bevorzugt stellen Pseudohalogene Thiocyanat, Cyanat, Isothiocyanat, Isocyanat und Azid dar.

Das erfindungsgemäße Verfahren zur Herstellung der Verbindungen der Formel (I) kann in Gegenwart eines Lösungsmittels oder in Substanz durchgeführt werden. Bevorzugt wird das Verfahren in Substanz durchgeführt. Beispielsweise und vorzugsweise können als Lösungsmittel die eingesetzten Edukte dienen oder lineare, zyklische und verzweigte Kohlenwasserstoffe, wie beispielsweise Hexane, Heptane und Octane, aromatische Kohlenwasserstoffe, wie beispielsweise Benzol, Toluol, Xylole, Ethylbenzol, Mesitylen, Ether wie beispielsweise 1,4-Dioxan, Tetrahydrofuran, Methyltetrahydrofuran, Dibutylether, Methyl-t-butylether, Diisopropylether, Diethylenglycol-dimethylether, Ester wie beispielsweise Ethylacetat, Butylacetat, Amide wie beispielsweise Dimethylformamid, Diethylformamid, N-Methylpyrrolidon, Dimethylacetamid oder Dimethylsulfoxid oder Sulfolan oder Nitrile wie beispielsweise Acetonitril, Isobutyronitril, Propionitril oder Propylencarbonat oder chlorierte aliphatische und aromatische Kohlenwasserstoffe oder Gemische dieser Lösungsmittel eingesetzt werden. Besonders bevorzugt werden Dimethylformamid, Diethylformamid, N-Methylpyrrolidon, Mesitylen, Dimethylacetamid, Dimethylsulfoxid, Sulfolan, Acetonitril und Propylencarbonat oder Gemische dieser Lösungsmittel eingesetzt. Ganz besonders bevorzugt werden N-Methylpyrrolidon, Mesitylen oder Gemische dieser beiden Lösungsmittel eingesetzt.

Das erfindungsgemäße Verfahren zur Herstellung der Verbindungen der Formel (I) wird beispielsweise bei Temperaturen zwischen 100°C und 300 °C, bevorzugt bei Temperaturen zwischen 140 °C und 250 °C, besonders bevorzugt bei Temperaturen zwischen 160 ° und 210 °C durchgeführt.

Das erfindungsgemäße Verfahren wird in der Regel bei Normaldruck durchgeführt. Im Allgemeinen kann das Verfahren bei jedem beliebigen Druck durchgeführt werden.

Gemäß dem erfindungsgemäßen Verfahren werden die Kupfer- und Palladiumquellen und die zyklischen, chelatisierenden Amine unabhängig voneinander in Mengen von 0,001 mol% bis 100 mol% bezogen auf die Verbindungen der Formel (III) eingesetzt, bevorzugt werden Mengen von 0,001 mol% bis 10 mol% eingesetzt und besonders bevorzugt werden Mengen von 0,01 mol% bis 6 mol% bezogen auf die Verbindungen der Formel (III) eingesetzt

Die Kupferquellen und die Palladiumquellen werden zudem bevorzugt in einem Stoffmengenverhältnis von 10(Kupferquelle):1(Palladiumquelle) bis 1:2, besonders bevorzugt in einem Stoffmengenverhältnis von 10:1 bis 1:1 eingesetzt.

In dem erfindungsgemäßen Verfahren zur Herstellung der Verbindungen der Formel (I) liegen die Stoffmengenverhältnisse der Verbindungen der Formel (II) und der Verbindungen der Formel (III) zwischen 4:1 und 1:10, bevorzugt zwischen 2:1 und 1:5 und besonders bevorzugt liegen die Stoffmengenverhältnisse der Verbindungen der Formel (II) und der Verbindungen der Formel (III) zwischen 2:1 und 1:2.

Die Phosphinliganden oder/und die zyklischen, chelatisierenden Amine können ebenfalls in Form von Komplexverbindungen der Kupfer- oder/und Palladiumquellen hinzugegeben werden. In diesem Fall ist eine zusätzliche Zugabe von Phosphinliganden oder/und zyklischen, chelatisierenden Amine nicht zwingend erforderlich. Diese Form der Reaktionsführung ist ebenfalls von dem erfindungsgemäßen Verfahren umfasst.

Das erfindungsgemäße Verfahren zur Herstellung der Verbindungen der Formel (I) aus den Verbindungen der Formel (II) wird im Wesentlichen wasserfrei durchgeführt. Im Wesentlichen wasserfrei bedeutet, dass der Wassergehalt bezogen auf die Menge der eingesetzten Reaktionsmischung vorzugsweise zwischen 0.0001 Gew. % und 1,0 Gew.% liegt.

In der Regel wird das erfindungsgemäße Verfahren zur Herstellung der Verbindungen der Formel (I) so durchgeführt, dass zunächst die Kupferquelle, das zyklische, chelatisierende Amin, die Palladiumquelle, der Phosphinligand und die Verbindungen der Formel (II) vorgelegt und dann das Lösungsmittel hinzugegeben wird. Danach wird die Reaktionsmischung im Allgemeinen inertisiert. Dann werden z.B. die Verbindungen der Formel (III) hinzugegeben und die Reaktionsmischung erwärmt. Das Ende der Reaktion kann mit dem Fachmann bekannten analytischen Verfahren, wie z.B. chromatographisch bestimmt werden. Die Aufarbeitung der Produkte erfolgt ebenfalls nach dem Fachmann bekannten und gängigen Verfahren, wie z.B mittels Extraktion mit organischen Lösungsmitteln oder/und Destillation. Die oben dargestellte Zugabe der Edukte kann ebenfalls in anderer Reihenfolge erfolgen.

Vorzugsweise wird das erfindungsgemäße Verfahren zur Herstellung der Verbindungen der Formel (I) so durchgeführt, dass zunächst die Kupferquelle, das zyklische, chelatisierende Amin, die Palladiumquelle und die Verbindungen der Formel (II) vorgelegt und dann das Lösungsmittel bzw. das Lösungsmittelgemisch hinzugegeben wird. Vorzugsweise wird dann die Reaktionsmischung inertisiert, z.B. durch Spülung mit Stickstoff. Dann werden die Verbindungen der Formel (III) hinzugegeben und die Reaktionsmischung erwärmt. Das Ende der Reaktion wird mittels Gaschromatographie bestimmt. Die Aufarbeitung der Produkte erfolgt ebenfalls nach dem Fachmann bekannten und gängigen Verfahren, wie z.B mittels Extraktion mit organischen Lösungsmitteln oder/und Destillation.

Von der Erfindung ist zudem ein Verfahren zur Herstellung der Verbindungen der Formel (II), 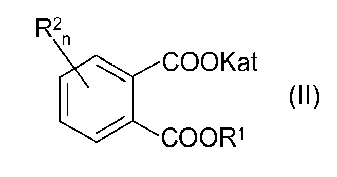 umfasst, wobei R¹ für C₁-C₆-Alkyl, C₆-C₂₄-Aryl oder C-₇-C₁₅-Arylalkyl steht und
R² unabhängig voneinander oder gleich sein kann und für C₁-C₆-Alkyl, C₂-C₆-Alkenyl, C₃-C₁₆-Heteroaryl, C₁-C₆-Alkoxy, C₁-C₆-Aryloxy, C₁-C₆-Acyl, C-₇-C₁₅-Arylalkyl, C₁-C₈-Mono- und Dialkylamino, Nitro, Cyano, C₁-C₆-Alkylthio, C₆-C₂₄-Aryl, 3- bis 7-gliedriger gesättigter oder teilweise ungesättigter Heterozyklus oder Wasserstoff steht und im Falle das R² ein C₃-Heteroaryl darstellt, dass C₃-Heteroaryl drei Kohlenstoffatome und mindestens zwei Stickstoffatome oder ein Stickstoffatom und ein Sauerstoffatom oder ein Stickstoffatom und ein Schwefelatom enthält und n = 1, 2, 3 oder 4 ist und Kat ein anorganisches oder organisches einfach geladenes Kation darstellt, wobei Verbindungen der Formel (V) 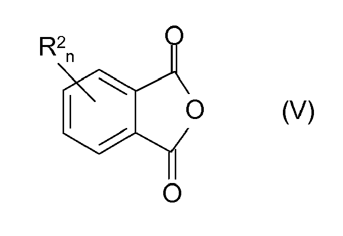 worin R² und n die vorgenannte Bedeutung besitzen,
in einem Schritt a) mit einer Verbindung der Formel (VI)

R¹(OH)ₘ, (VI)

wobei R¹ die vorgenannte Bedeutung besitzt und m = 1,2,3 oder 4 sein kann,
zu Verbindungen der Formel (VII) 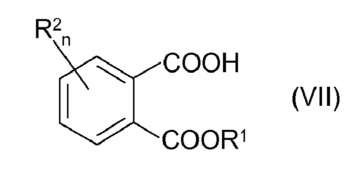 und in einem Schritt b) die Verbindungen der Formel (VII) mit mindestens einer Base, bestehend aus einem oder mehreren einfach geladenen, anorganischen oder organischen Kationen und korrespondierenden Anionen, zu Verbindungen der Formel (II) umgesetzt werden.

Basen im Sinne des erfindungsgemäßen Verfahrens zur Herstellung der Verbindungen der Formel (II) aus den Verbindungen der Formel (V) stellen vorzugsweise Alkalimetallhydroxide oder -alkylate, wie z.B. - Natriummethylat, Kalium-tert.-butylat, Natriumhydroxid, Kaliumhydroxid oder organische Basen, wie z.B. Pyridin, Ammmonmiumverbindungen, wie z.B. Ammoniumhydroxid oder Phosphoniumverbindungen oder Mischungen dieser Basen dar. Besonders bevorzugt wird als Base Kaliumhydroxid eingesetzt. Kat stellt im Sinne des erfindungsgemäßen Verfahrens zur Herstellung der Verbindungen der Formel (II) aus den Verbindungen der Formel (V) das zu diesen Basen korrespondierende Kation dar.

Bevorzugt ist m = 1 oder 2, besonders bevorzugt ist m = 1.

Verbindungen der Formel (VI) im Sinne der Erfindung stellen ein-, zwei-, oder dreiwertige aliphatische oder aromatische Alkohole dar. Bevorzugt werden als Verbindungen der Formel (VI) aliphatische, zyklische oder nichtzyklische verzweigte oder unverzweigte Alkohole eingesetzt. Besonders bevorzugt werden als Verbindungen der Formel (VI) Benzol, Phenol, Methanol, Ethanol, n- oder iso-Propanol, n-, s-, i,- oder tert.-Butanol, neo-Pentanol oder cyclo-Hexanol eingesetzt, ganz besonders bevorzugt wird als Verbindungen der Formel (VI) iso- bzw. s-Propanol eingesetzt.

Das erfindungsgemäße Verfahren zur Herstellung der Verbindungen der Formel (II) kann in Gegenwart eines organischen Lösungsmittels durchgeführt werden. Vorzugsweise wird das Verfahren in Substanz durchgeführt. Besonders bevorzugt werden die Verbindungen der Formel (VI) soweit im Überschuss eingesetzt, dass die Verbindungen der Formel (VI) als Lösungsmittel und als Edukte verwendet werden können. Falls das Verfahren in Gegenwart eines organischen Lösungsmittels durchgeführt wird, ist jedes organische, inerte Lösungsmittel geeignet mit dem sich die Verbindungen der Formel (VI) mischen lassen. Als organisches, inertes Lösungsmittel im erfindungsgemäßen Verfahren eignen sich insbesondere polare, organische Lösungsmittel, wie z.B. Ketone, Nitrile oder Sulfone.

Schritt a) des erfindungsgemäßen Verfahrens zur Herstellung der Verbindungen der Formel (II) erfolgt beispielsweise und vorzugsweise bei Temperaturen bei denen die Verbindungen der Formel (VI) sieden. Schritt a) des erfindungsgemäßen Verfahrens kann bei verschiedenen Drücken durchgeführt werden. Bevorzugt wird Schritt a) des erfindungsgemäßen Verfahrens zur Herstellung der Verbindungen der Formel (II) bei Standarddruck durchgeführt.

Die in Schritt a) des erfindungsgemäßen Verfahrens zur Herstellung der Verbindungen der Formel (II) eingesetzten Verbindungen der Formel (VI) werden beispielsweise und vorzugsweise im molaren Überschuss zu den Verbindungen der Formel (V) eingesetzt. Bevorzugt werden die Verbindungen der Formel (V) und die Verbindungen der Formel (VI) in einem Stoffmengenverhältnis größer als 1:5 eingesetzt, besonders bevorzugt in einem Stoffmengenverhältnis größer als 1:10 eingesetzt.

Die in Schritt b) des erfindungsgemäßen Verfahrens zur Herstellung der Verbindungen der Formel (II) eingesetzten Basen und die Verbindungen der Formel (VII) werden beispielsweise in einem Stoffmengenverhältnis von 0,1 bis 10, bevorzugt von 0,1 bis 5 und besonders bevorzugt in einem Stoffmengenverhältnis von 0,8 bis 1,2 eingesetzt.

Schritt b) des erfindungsgemäßen Verfahrens zur Herstellung der Verbindungen der Formel (II) kann beispielsweise bei Raumtemperatur unter Standardruck durchgeführt werden.

Schritt a) des erfindungsgemäßen Verfahrens zur Herstellung der Verbindungen der Formel (II) wird vorzugsweise so durchgeführt, dass die Verbindungen der Formel (V) mit den Verbindungen der Formel (VI) vermischt werden und danach erhitzt wird. Das Ende der Reaktion kann gaschromatographisch bestimmt werden. Dann werden die Verbindungen der Formel (VII) isoliert und weiter zu den Verbindungen der Formel (II) umgesetzt. Dies geschieht, indem Verbindungen der Formel (VII) in einem Lösungsmittel gelöst werden und dazu, bevorzugt dosiert, die Basen hinzugegeben werden. Auch andere Zugabereihenfolgen sind prinzipiell möglich.

In einer besonderen Ausführungsform der Erfindung erfolgt die erfindungsgemäße Herstellung der Verbindungen der Formel (II) in einem Eintopfverfahren, insbesondere ohne weitere Aufreinigung der Verbindungen der Formel (VII). Dies bedeutet, dass der Schritt b) in Schritt a) durchgeführt wird und demzufolge Schritt b) entfallen kann.

In einer weiteren besonders bevorzugten Ausführungsform erfolgt die Herstellung der Verbindungen der Formel (I) in einem Eintopfverfahren, insbesondere ohne weitere Aufreinigung der Verbindungen der Formel (II). In dieser Ausführungsform werden im Allgemeinen zunächst die Verbindungen der Formel (V) vorgelegt. Danach können die Verbindungen der Formel (VI) zugegeben. Das Ende der Reaktion kann z.B. mittels Gaschromatographie bestimmt. Danach wird beispielsweise die Base hinzugegeben. Nach dem Ende der Reaktion wird z.B. das entstandene Wasser, beispielsweise durch Destillation abgetrennt. Danach kann beispielsweise das Lösungsmittel, die Kupferquelle, das zyklische, chelatisierende Amin, der Phosphinligand, die Palladiumquelle und dann die Verbindungen der Formel (III) hinzugegeben werden. Dann kann beispielsweise inertisiert werden, z.B. durch Durchspülung der Reaktionsmischung mit Stickstoff oder Argon. Danach könnte beispielsweise erwärmt werden. Das Ende der Reaktion kann z.B. mittels Gaschromatographie bestimmt werden. Die Aufarbeitung erfolgt nach gängigen, dem Fachmann bekannten Verfahren, z.B. mittels Extraktion oder Destillation.

Vorzugsweise werden die Verbindungen der Formel (V) vorgelegt und die Verbindungen der Formel (VI) zugegeben. Das Ende der Reaktion wird nach dem Fachmann bekannten analytischen Wegen bestimmt. Dann wird die Base hinzugegeben und das entstehende Wasser, bevorzugt destillativ, entfernt. Dann werden die Lösungsmittel die Verbindungen der Formel (III), die Kupferquelle, das zyklische, chelatisierende Amin, der Phosphinligand und die Palladiumquelle hinzugegeben, inertisiert und danach erwärmt. Das Ende der Reaktion wird gaschromatographisch bestimmt.

Falls das erfindungsgemäße Verfahren zur Herstellung der Verbindungen der Formel (II) in einem Eintopfverfahren durchgeführt wird, liegen die Stoffmengenverhältnis der Verbindungen der Formel (V) und der Basen beispielsweise zwischen 0,1 bis 10, bevorzugt zwischen 0,1 bis 5 und besonders bevorzugt zwischen 0,8 bis 1,2.

Die Verbindungen der Formel (I) stellen 4'-Halogenalkyl-biphenyl-2-carbonsäureester dar. Aus den Verbindungen der Formel (I) lassen sich durch Verseifung mit einer Säure oder einer Base die Verbindungen der Formel (VIII) 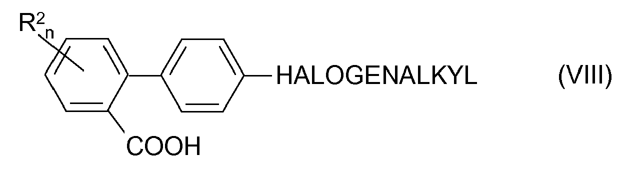 in denen R², n und HALOGENALKYL die zu den Verbindungen der Formel (I) angegebene Bedeutung haben, herstellen.

Von der Erfindung ist daher auch ein Verfahren zur Herstellung der Verbindungen der Formel (VIII) aus den Verbindungen der Formel (VIII) umfasst. Die Verbindungen der Formel (VIII) können ebenfalls in Form ihrer Salze vorliegen bzw. in Form dieser Salze aus den Verbindungen der Formel (I) hergestellt werden. Als Säuren oder Basen für die Herstellung der Verbindungen der Formel (VIII) aus den Verbindungen der Formel (I) können Schwefelsäure, Salpetersäure, Halogenwasserstoffsäuren oder Alkalimetallhydroxide eingesetzt werden.

Die Herstellung der Verbindungen der Formel (VIII) aus den Verbindungen der Fomel (I) kann bei Temperaturen zwischen 20 °C bis 200 °C, bevorzugt zwischen 30 °C und 100 °C durchgeführt werden.

Als Lösungmittel für die Herstellung der Verbindungen der Formel (VIII) aus den Verbindungen der Fomel (I) können alle mit Wasser mischbaren organischen, inerten Lösungsmittel, wie z.B. inerte Amide, wie beispielsweise und vorzugsweise Dimethylformamid, Diethylformamid, N-Methylpyrrolidon, oder Dimethylacetamid eingesetzt werden.

Generell kann die Herstellung der Verbindungen der Formel (VIII) aus den Verbindungen der Formel (I) nach gängigen dem Fachmann bekannten Bedingungen für die Verseifung eines Carbonsäureesters durchgeführt werden.

Die in den erfindungsgemäßen Verfahren verwendeten Einsatzstoffe und Edukte sind entweder nach dem Fachmann bekannten Verfahren herstellbar oder kommerziell erhältlich.

Mit Hilfe des erfindungsgemäßen Verfahrens können die Verbindungen der Formel (I) in hohen Ausbeuten und ökonomisch in technischen Prozessen hergestellt werden. Die entstehenden Nebenprodukte sind aus ökologischer Sicht wenig bedenklich. Zudem weist das erfindungsgemäße Verfahren unter sicherheitstechnischen Aspekten Vorteile auf. Das Verfahren zur Herstellung der Verbindungen der Formel (I) lässt sich besonders effizient in einem Eintopfverfahren, ohne weitere Aufreinigung der Verbindungen der Formel (II) oder der Verbindungen der Formel (VII), mit Verbindungen der Formel (V) als Edukte durchführen. Die Verbindungen der Formel (I) lassen sich durch Verseifung leicht in die Verbindungen der Formel (VIII) überführen, die sich so bei Bedarf herstellen lassen.

Die Verbindungen der Formel (VIII) und damit auch die Verbindungen der Formel (I) stellen wichtige Zwischenprodukte für pharmazeutische Wirkstoffe, die z.B. für die Behandlung von Herz-Kreislauferkrankungen geeignet sind. Einige der Verbindungen der Formel (VIII), insbesondere 4'-Trifluormethyl-biphenyl-2-carbonsäure, sind als Arzneimittel geeignet.

Die folgenden Beispiele dienen der beispielhaften Erläuterung der Erfindung und sind nicht als Beschränkung aufzufassen.

### Beispiele

### 1. Herstellung von Phthalsäuremonoisopropylester

Phthalsäureanhydrid (14.8 g, 100 mmol) wurde in einem 100 mL Rundkolben mit Rückflusskühler in ⁱPrOH (iso-Propanol) (50 mL) suspendiert und 6h zum Sieden erhitzt. Nach Abkühlen der klaren Lösung auf Raumtemperatur wurde eingeengt und der verbliebene farblose ölige Rückstand wurde i. Vak. getrocknet, wobei dieser langsam auskristallisierte und das Produkt (20.8 g, 99%) als farbloser Feststoff erhalten wurde. ¹H-NMR (600 MHz, CDCl₃) δ = 12.46 (s, 1 H), 7.85 (d, ³*J*= 7.6 Hz, 1 H), 7.64 (d, ³*J*= 7.6 Hz, 1 H), 7.54 (t, ³*J*= 7.6 Hz, 1 H), 7.49 (t, ³*J*= 7.6 Hz, 1 H), 5.24 (h, ³*J*=6.2 Hz, 1 H), 1.32 (d, ³*J*= 6.7 Hz, 6 H) ppm. ¹³C-NMR (151 MHz, CDCl₃) δ = 172.6, 167.5, 133.7, 132.0, 130.4, 129.6, 129.5, 128.5, 69.5, 21.3 ppm.

### 2. Herstellung von Kaliummonoisopropylphthalat

Phthalsäuremonoisopropylester (10.4 g, 50.0 mmol) wurde in einem 250 mL Rundkolben in 2-Propanol (20 mL) gelöst. Eine Lösung von Kaliumhydroxid (3.30 g, 50.0 mmol) in 2-Propanol wurde innerhalb von 30 min bei Raumtemperatur zugetropft. Nach beendeter Zugabe wurde eine weitere halbe Stunde gerührt, dann das Lösungsmittel entfernt und der verbliebene farblose Feststoff in Methyl-tert.-Butylether (100 mL) aufgenommen. Die Suspension wurde 15 min gerührt, wobei ein voluminöser farbloser Feststoff ausfiel, der abfiltriert, mit Methyl-tert.-Butylether (3 × 50 mL) gewaschen und i. Vak. getrocknet wurde. Das Salz (9.82 g, 80%) wurde als farbloser Feststoff erhalten. ¹H-NMR (400 MHz, D₂O δ = 7.64 (d, ³*J*= 7.5 Hz, 1 H), 7.42―7.52 (m, 2 H), 7.36 (t, ³*J*= 7.5 Hz, 1 H), 5.09 (h, ³*J*= 6.2 Hz, 1 H), 1.29 (d, ³*J*= 6.2 Hz, 6 H) ppm. ¹³C NMR (101 MHz, D₂O) δ = 176.6, 169.9, 141.0, 132.3, 129.2, 128.8, 128.8, 127.4, 70.8, 21.3 ppm.

### 3. Herstellung von Kaliummononeopentylphthalat

Neopentylalkohol (25.0 g, 284 mmol) wurde in einem 250 mL Rundkolben eingewogen, auf 70 °C erwärmt wobei der Alkohol schmolz. Kalium-*tert*-butylat (5.61 g, 50.0 mmol) wurde hinzugeben und so lange gerührt bis eine klare, farblose Lösung erhalten wurde. Phthalsäureanhydrid (7.41 g, 50.0 mmol) wurde zugegeben, 10 min gerührt und die farblose, schwer rührbare Reaktionsmasse wurde auf Raumtemperatur gekühlt, in Ethanol suspendiert und mit Ethanol gewaschen, filtriert und im Vakuum getrocknet. Das Produkt (13.1 g, 95%) wurde als farbloser Feststoff erhalten. ¹H-NMR (400 MHz, MeOH-D4) δ = 7.84―7.86 (m, 1 H), 7.59―7.62 (m, 2 H), 7.47―7.52 (m, 1 H), 4.08 (s, 2 H), 1.13 (s, 9 H) ppm. ¹³C NMR (101 MHz, MeOH-*D*4) δ = 176.8, 170.0, 143.5, 132.8, 130.5, 130.0, 129.1, 128.7, 76.0, 32.3, 27.2 ppm.

### 4. Herstellung von Kaliummonobenzylphthalat

In einem 500 mL Rundkolben wurde Kalium-*tert*-butylat (11.2 g, 100 mmol), in Benzylalkohol (50.0 g, 462 mmol) bei 70°C gelöst. Zu der klaren, farblosen Lösung wurde gepulvertes Phthalsäureanhydrid (14.8 g, 100 mmol) zugegeben und 1 h bei 70 °C gerührt. Das Lösungsmittel wurde entfernt und der verbliebene farblose, wachsige Rückstand wurde mit Ethanol mehrfach gewaschen und im Vakuum getrocknet. Das Produkt (28.9 g, 98%) wurde als farbloser Feststoff erhalten. ¹H-NMR (400 MHz, DMSO-*D*6) δ = 7.72 (d, ³*J*= 7.5 Hz, 1 H), 7.44 (d, ³*J*= 7.2 Hz, 2 H), 7.27―7.39 (m, 6 H), 5.21 (s, 2 H) ppm. ¹³C NMR (101 MHz, DMSO-*D*6) δ = 170.2, 169.0, 141.1, 136.5, 133.0, 129.2, 128.7, 128.3, 127.9, 127.7, 127.5, 126.2, 66.0 ppm.

### 5. Herstellung von 4'-Trifluormethylbiphenyl-2-carbonsäureisopropylester

In einem 20 ml Headspacevial wurden Kupfer(I)chlorid (5.94 mg, 0.06 mmol), 1,10-Phenanthrolin (10.8 mg, 0.06 mmol), Pd(acac)₂ (Palladium(II)acetylacetonat) (1.83 mg, 0.06 mmol), 2-(Di-*tert-*butyl-phosphino)-biphenyl (John-Phos) (5.37 mg, 0.018 mmol) und Kaliummonoisopropylphthalat (493 g, 2.00 mmol) eingewogen, 30min i. Vak. getrocknet, mit Stickstoff rückbefüllt, eine frisch entgaste Lösung von Mesitylen und N-Methylpyrrolidon (4.0 mL, 3:1) und 4-Chlorbenzotrifluorid (361 g, 2.00 mmol, 2677 µL) zugegeben. Die orange Reaktionsmischung wurde 16h bei 170 °C gerührt. Nach Abkühlen auf Raumtemperatur wurde die rot-braune Reaktionslösung filtriert und mit Ethylacetat und danach mit wässr. HCl gewaschen, erneut mit Ethylacetat extrahiert und danach getrocknet. Das verbliebene braune Öl wurde mittels Flashchromatographie (Kieselgel, *n-*Hexan/Ethylacetat, 9:1) gereinigt und das Produkt (409 mg, 66%) als farbloses Öl erhalten. ¹H-NMR (600 MHz, CDCl₃) δ = 7.92 (d, ³*J*= 7.9 Hz, 1 H), 7.66 (d, ³*J*= 8.2 Hz, 2 H), 7.52 (t, ³*J*= 7.4 Hz, 1 H), 7.40―7.46 (m, 3 H), 7.31 (d, ³*J*= 7.7 Hz, 1 H), 5.01 (h, ³*J*= 6.2 Hz, 1 H), 1.04 (d, ³*J*= 6.2 Hz, 6 H) ppm. ¹³C-NMR (151 MHz, CDCl₃) δ = 167.4, 145.4, 140.9, 131.3, 131.1, 130.3, 129.9, 129.3, 129.1, 128.8, 127.7, 125.1, 124.7 (q), 123.3, 68.5, 21.1 ppm.

### 6. Herstellung von 4'-Trifluormethylbiphenyl-2-carbonsäureisopropylester in einem Eintopfverfahren

Phtalsäureanhydrid (35,4 g; 0,24 mol) wurde in einem 0,5 L Sulfierbecher mit KPG-Rührer, Kombikühler (wahlweise als Intensivkühler und als Destillationsbrücke einsetzbar) und Tropftrichter in 120 mL Isopropanol suspendiert und anschließend für 6 h bei 60 °C gerührt. Die entstehende Lösung wurde auf Raumtemperatur abgekühlt und eine Lösung von KOH in Isopropanol (15,8 g in 120 mL) zugegeben und für eine weitere Stunde gerührt. Anschließend wurde N-Methylpyrrolidon zugegeben und Isopropanol und das enstandene Wasser destillativ entfernt. Die erhaltende Lösung wurde mit 270 mL Mesitylen, 28,8 g 4-Chlorbenzotrifluorid, 0,8 g CuCl, 1,6 g 1,10-Phenanthrolin, 0,8 g Palladiumacetat und 2,2 g Dicyclohexylphosphin-2-biphenyl (Dicyclohexyl-Johnphos) versetzt. Die Reaktionsmischung wurde evakuiert und anschließend mit Stickstoff belüftet und auf 170 °C erwärmt. Die Reaktionslösung wurde dann für 24 h bei 170 °C gerührt abgekühlt und über Aktivkohle filtriert. Anschließend wurde zunächst Mesitylen abdestilliert und der Rückstand in Isopropylacetat aufgenommen. Die organische Phase wurde mit 10%iger Salzsäure versetzt, extrahiert und die Phase getrennt. Von der organischen Phase wurden zunächst die Lösungsmittel unter vermindertem Druck destillativ entfernt anschließend auch das Produkt destilliert. Man erhielt 34,4 g (0,11 mol, 70%) 4'-Trifluormethylbiphenyl-2-carbonsäureisopropylester als farbloses Öl. 1H-NMR (400 MHz, CDCl3) δ = 7.92 (d, 3J = 7.9 Hz, 1 H), 7.66 (d, 3J = 8.2 Hz, 2 H), 7.52 (t, 3J = 7.4 Hz, 1 H), 7.40―7.46 (m, 3 H), 7.31 (d, 3J = 7.7 Hz, 1 H), 5.01 (h, 3J = 6.2 Hz, 1 H), 1.04 (d, 3J = 6.2 Hz, 6 H) ppm.

### 7. Herstellung von 4'-Trifluormethylbiphenyl-2-carbonsäureisopropylester

In einem ofengetrocknetem 250 mL Dreihalskolben mit Rückflusskühler wurde Kaliummonoisopropylphthalat (26.6 g, 108 mmol), Kupfer(II)oxid (358 mg, 4.50 mmol), 1,10-Phenanthrolin (811 mg, 4.50 mmol), Pd(acac)₂ (137 mg, 0.45mmol) und Cyclohexyl-John-Phos (315 mg, 0.90 mmol) eingewogen und 1 h i. Vak. getrocknet. Die Apparatur wurde mit Stickstoff rückbefüllt, eine mit Argon entgaste Lösung aus trockenem Mesitylen/NMP (180 mL, 3:1) und 4-Chlorbenzotrifluorid (16.6 g, 92.2 mmol, 12.3 mL) wurden zugegeben. Die grau-schwarze Suspension wurde bei 180°C Ölbadtemperatur 16 h gerührt. Nach Abkühlen auf Raumtemperatur wurde die Apparatur mit Ethylacetat (50 mL) gespült und die rötlich-braune Reaktionsmischung durch Celite (15 g), das mit Kieselgel (20 g) bedeckt war, filtriert. Der Filterkuchen wurde mit Ethylacetat (3 × 100 mL) gespült und die vereinigten Filtrate wurden mit 1N Salzsäure (3 × 450 mL) gewaschen. Die wässrige Phase wurde mit Ethylacetat (2 × 100 mL) reextrahiert. Die vereinigten organischen Phasen wurden mit gesättigter Kochsalzlösung (1 × 150 mL) gewaschen, über MgSO₄ getrocknet, filtriert und konzentriert und fraktioniert Vakuum destilliert und Teilfraktionen Säulenchromatographisch aufgereinigt. 4'-Trifluormethyl-2-biphenylcarbonsäureisopropylester konnte in einer Ausbeute von 79% isoliert werden. Sdp.: 98―99 °C/3 × 10⁻³ mbar. ¹H-NMR (600 MHz, CDCl₃) δ = 7.92 (d, ³*J*= 7.9 Hz, 1 H), 7.66 (d, ³*J*= 8.2 Hz, 2 H), 7.52 (t, ³*J*= 7.4 Hz, 1 H), 7.40―7.46 (m, 3 H), 7.31 (d, ³*J*= 7.7 Hz, 1 H), 5.01 (h, ³*J*= 6.2 Hz, 1 H), 1.04 (d, ³*J*= 6.2 Hz, 6 H) ppm. ¹³C-NMR (151 MHz, CDCl₃) δ = 167.4, 145.4, 140.9, 131.3, 131.1, 130.3, 129.9, 129.3, 129.1, 128.8, 127.7, 125.1, 124.7 (q), 123.3, 68.5, 21.1 ppm. MS (Ion trap, EI): m/z (%) = 308 [M⁺] (22), 265 (100), 249 (45), 201 (41), 152 (21), 43 (12).

### 8. Herstellung von 4'-Trifluormethylbiphenyl-2-carbonsäure

In einem 0,5 L Sulfierbecher mit KPG-Rührer und Rückflusskühler wurden 50,7 g 4-Trifluormethylbiphenyl-2-carbonsäureisopropylester (0,165 mol) in 200 g N-Methylpyrrolidon bei Raumtemperatur vorgelegt und mit 196 g einer 20%igen Kalilauge versetzt. Der Reaktionsansatz wurde über Nacht bei 90 bis 95 °C gerührt. Anschließend wurde auf Raumtemperatur abgekühlt und mit 300 ml einer 20%igen Salzsäure auf pH 1-2 angesäuert. Die Reaktionssupension wurde auf 10 °C gekühlt und 3 h nachgerührt. Vom Niederschlag wurde abfiltriert und der Filterrückstand mit viel Wasser gewaschen. Das Feuchtprodukt wurde anschließend im Vakuumtrockenschrank über Nacht bei 40 °C und 20 mbar getrocknet. Man erhielt einen 31,7 g 4-Trifluormethylbiphenyl-2-carbonsäure (0,119 mol, 72%) als farblosen Feststoff.

## Patentansprüche

1. Verfahren zur Herstellung der Verbindungen der Formel (I) 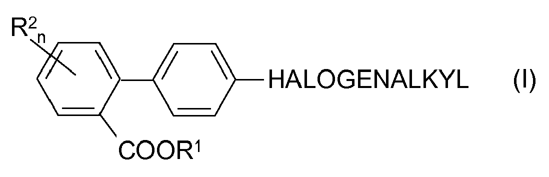 wobei R¹ für C₁-C₆-Alkyl, C₆-C₂₄-Aryl oder C₇-C₁₅-Arylalkyl steht und R² unabhängig voneinander oder gleich sein kann und für C₁-C₆-Alkyl, C₂-C₆-Alkenyl, C₃-C₁₆-Heteroaryl, C₁-C₆-Alkoxy, C₁-C₆-Aryloxy, C₁-C₆-Acyl, C-,-C₁₅-Arylalkyl, C₁-C₈-Mono- und Dialkylamino, Nitro, Cyano, C₁-C₆-Alkylthio, C₆-C₂₄-Aryl, einen 3- bis 7-gliedrigen gesättigten oder teilweise ungesättigten Heterozyklus oder Wasserstoff steht und im Falle, dass R² ein C₃-Heteroaryl darstellt, C₃-Heteroaryl drei Kohlenstoffatome und mindestens zwei Stickstoffatome oder ein Stickstoffatom und ein Sauerstoffatom oder ein Stickstoffatom und ein Schwefelatom enthält,
und n = 1, 2, 3 oder 4 ist,
und HALOGENALKYL für einen einfach, mehrfach oder vollständig halogenierten, also durch F, Cl, Br, oder/und I substituierten, geradkettigen, zyklischen oder verzweigten C₁-C₆-Alkylrest steht,
bei dem Verbindungen der Formel (II) 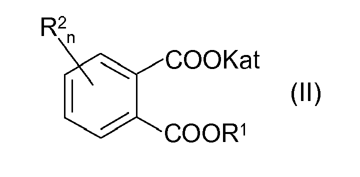 worin R¹, R² und n die vorgenannte Bedeutung besitzen und Kat ein anorganisches oder organisches einfach geladenes Kation darstellt,
in Gegenwart mindestens einer Kupferquelle,
in Gegenwart von mindestens einem zyklischen, chelatisierenden Amin in Gegenwart von mindestens einem Phosphinliganden und
in Gegenwart mindestens einer Palladiumquelle
mit Verbindungen der Formel (III) 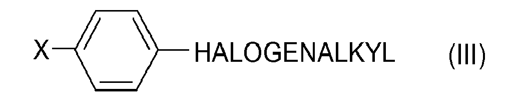 in denen HALOGENALKYL die vorgenannte Bedeutung besitzt und X für Halogen = Cl, Br, I, Pseudohalogen, Arylsulfonat oder Alkylsulfonat steht, zu Verbindungen der Formel (I) umgesetzt werden.

2. Verfahren nach Anspruch 1, **dadurch gekennzeichnet, dass** R¹ Methyl, Ethyl, s-Propyl, n-Propyl, n-, s-, i-, tert-Butyl, neo-Pentyl, cyclo-Hexyl, Benzyl oder Phenyl ist.

3. Verfahren nach Anspruch 1 oder 2, **dadurch gekennzeichnet, dass** R² für Wasserstoff oder/und für C₁-C₆-Alkyl, C₂-C₆-Alkenyl, C₃-C₁₆-Heteroaryl, C₆-C₂₄-Aryl , C₁-C₆-Acyl steht.

4. Verfahren nach einem oder mehreren der Ansprüche 1 bis 3, **dadurch gekennzeichnet, dass** HALOGENALKYL für ein C₁-C₆-Perhalogenalkyl, bevorzugt für ein C₁-C₆-Perfluoralkyl, steht.

5. Verfahren nach einem oder mehreren der Ansprüche 1 bis 4 **dadurch gekennzeichnet, dass** die Kupferquelle eine anorganische Kupfer(I)- oder Kupfer (II)verbindung, bevorzugt Kupfer(I)halogenide, Kupfer(II)halogenide oder Kupfer(II)oxid darstellt.

6. Verfahren nach einem oder mehreren der Ansprüche 1 bis 5, **dadurch gekennzeichnet, dass** als Palladiumquelle ein organisches Palladium(II)salz, bevorzugt Palladium(II)acetylacetonat, eingesetzt wird.

7. Verfahren nach einem oder mehreren der Ansprüche 1 bis 6 **dadurch gekennzeichnet, dass** als zyklisches, chelatisierendes Amin ein zyklisches, aromatisches Diamin eingesetzt wird.

8. Verfahren nach einem oder mehreren der Ansprüche 1 bis 7, **dadurch gekennzeichnet, dass** als Phosphinliganden Verbindungen der Formel (IV), 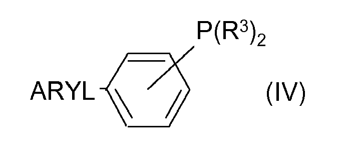 wobei ARYL einen einfach oder mehrfach unabhängig voneinander durch C₁-C₆-Alkyl, C₁-C₆-Alkoxy, Sulfonaten oder C₁-C₈-Mono- bzw. Dialkylamino substituierten oder unsubstituierten C₆-C₂₄-Aryl darstellt und R³ einen C₁-C₆-Alkylrest oder einen einfach oder mehrfach substituierten oder unsubstituierten C₆-C₂₄-Aryl darstellt, eingesetzt werden.

9. Verfahren nach einem oder mehreren der Ansprüche 1 bis 8, **dadurch gekennzeichnet**, X für Cl, Triflat, Tosylat, Nonaflat oder Mesylat steht.

10. Verfahren zur Herstellung der Verbindungen der Formel (II), 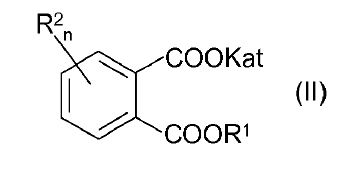 wobei R¹ für C₁-C₆-Alkyl, C₆-C₂₄-Aryl oder C-₇-C₁₅-Arylalkyl steht und
R² unabhängig voneinander oder gleich sein kann und für C₁-C₆-Alkyl, C₂-C₆-Alkenyl, C₃-C₁₆-Heteroaryl, C₁-C₆-Alkoxy, C₁-C₆-Aryloxy, C₁-C₆-Acyl, C₇-C₁₅-Arylalkyl, C₁-C₈-Mono-und Dialkylamino, Nitro, Cyano, C₁-C₆-Alkylthio, C₆-C₂₄-Aryl, einen 3- bis 7-gliedrigen gesättigten oder teilweise ungesättigten Heterozyklus oder Wasserstoff steht und im Falle, dass R² ein C₃-Heteroaryl darstellt, dass C₃-Heteroaryl drei Kohlenstoffatome und mindestens zwei Stickstoffatome oder ein Stickstoffatom und ein Sauerstoffatom oder ein Stickstoffatom und ein Schwefelatom enthält,
und n = 1, 2, 3 oder 4 ist
und Kat ein anorganisches oder organisches einfach geladenes Kation darstellt, **dadurch gekennzeichnet, dass** Verbindungen der Formel (V) 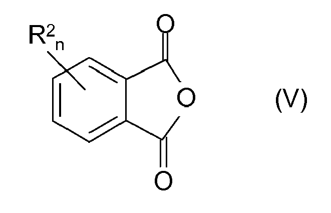 worin R² und n die vorgenannte Bedeutung besitzen,
in einem Schritt a) mit einer Verbindung der Formel (VI)
R¹(OH)" (VI)
wobei R¹ die vorgenannte Bedeutung besitzt und m = 1, 2, 3 oder 4 sein kann,
zu Verbindungen der Formel (VII) 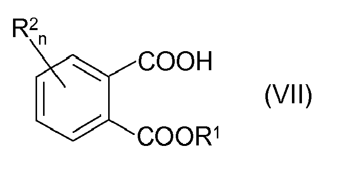 und in einem Schritt b) die Verbindungen der Formel (VII)
mit mindestens einer Base, bestehend aus einem oder mehreren einfach geladenen, anorganischen oder organischen Kationen und korrespondierenden Anionen, zu Verbindungen der Formel (II) umgesetzt werden.

11. Verfahren nach Anspruch 10, **dadurch gekennzeichnet, dass** als Basen Alkalimetallhydroxide, bevorzugt Kaliumhydroxid, eingesetzt werden.

12. Verfahren nach einem der Ansprüche 10 oder 11, **dadurch gekennzeichnet, dass** die Verbindungen der Formel (II) in einer Eintopfreaktion aus den Verbindungen der Formel (V) ohne Isolierung oder Aufreinigung der Verbindungen der Formel (VII) hergestellt werden.

13. Verfahren nach einem oder mehreren der Ansprüche 1 bis 9 , **dadurch gekennzeichnet, dass** die Verbindungen der Formel (II) nach einem oder mehreren der Ansprüche 10 bis 12 hergestellt werden und ohne Isolierung und weitere Aufreinigung eingesetzt werden.

14. Verfahren nach einem oder mehreren der Ansprüche 1 bis 9, **dadurch gekennzeichnet, dass** die Verbindungen der Formel (VIII) 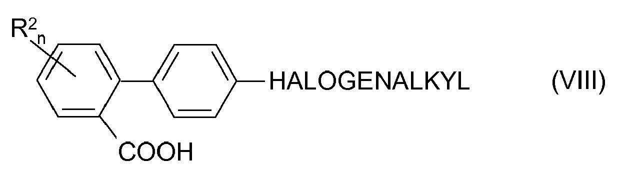 in denen R², n und HALOGENALKYL die zu den Verbindungen der Formel (I) angegebene Bedeutung haben, durch Umsetzung der Verbindungen der Formel (I) mit einer Säure oder einer Base hergestellt werden.
